# EUROPEAN PATENT APPLICATION

(11) **EP 3 845 883 A1**
(43) Date of publication of application: **07.07.2021**
(21) Application number: 19853366.3
(22) Date of filing: 19.08.2019
(51) Int. Cl.: G01N 1/04, D21H 11/00, G01N 33/48

(54) **FECES-RECEIVING SHEET FOR FECES COLLECTION**

(30) Priority: 31.08.2018 JP 2018163955
(71) Applicant: Nippon Paper Papylia Co., Ltd., Fuji-shi Shizuoka 417-0852 (JP)
(72) Inventor: KISHIMOTO, Masaki, Tokyo 101-0062 (JP); ISHINO, Yoshiaki, Tokyo 101-0062 (JP)
(74) Representative: Grünecker Patent- und Rechtsanwälte PartG mbB
(86) International application number: PCT/JP2019/032215
(87) International publication number: WO 2020/045137

(57) **Abstract**

An object is to provide a stool-receiving sheet for stool collection that offers good floatability and dispersibility on/in water. As a solution, a stool-receiving sheet for stool collection is provided whose compounding quantity of a softwood pulp with a lumen width (1) to fiber diameter (D) ratio (1/D) of 0.6 or lower is 40 percent by weight or higher, while compounding quantity of a sizing agent is 0.05 percent by weight or higher but no higher than 0.12 percent by weight, relative to the total quantity of papermaking fibers, and whose density is 0.60 g/cm³ or lower.

## Description

### Technical Field

The present invention relates to a stool-receiving sheet for stool collection that can be floated on standing water in a toilet bowl, etc., for stool collection, and is directly flushable once a sample has been collected.

### Background Art

Stool collection is widely practiced for testing digestive diseases, parasitic infections, bacterial infections, etc., in the large intestine, etc.

Stool collection, or collection of a stool sample, is generally performed by floating a stool-receiving sheet for stool collection on standing water in a flushing toilet bowl. This stool-receiving sheet for stool collection must be floatable on water so that it will remain floating on the water when a sample is collected, and also be dispersible in water so that it will not cause the drain pipe to clog when flushed directly in water after a sample has been collected.

Such stool-receiving sheets for stool collection include, for example: a stool-receiving sheet for stool collection proposed by Patent Literature 1, comprising a sheet body made of water-soluble paper whose solubility in water has been adjusted by treating at least one side for water resistance using a water resistant treatment solution; a stool-receiving sheet for stool collection proposed by Patent Literature 2, made of a paper of 0.7 g/cm³ or lower in density which contains wood pulp or other natural fibers and mercerized pulp or rayon or both, and to which a sizing agent has been added during wet papermaking; and a stool collection sheet proposed by Patent Literature 3, having a basis weight of 20 to 60 g/m² and whose stool retention property and dispersibility in water have been adjusted based on a combination of a sizing agent and a degree of beating of pulp.

### Background Art Literature

### Patent Literature

Patent Literature 1: Japanese Patent Laid-open No. Hei 10-68726
Patent Literature 2: Japanese Patent Laid-open No. 2004-150911
Patent Literature 3: Japanese Patent Laid-open No. 2006-98133

### Summary of the Invention

### Problems to Be Solved by the Invention

An object of the present invention is to provide a stool-receiving sheet for stool collection that offers good floatability and dispersibility on/in water.

### Means for Solving the Problems

The means provided by the present invention for achieving the object are as follows:
1. A stool-receiving sheet for stool collection, characterized in that: relative to the total quantity of papermaking fibers, the compounding quantity of a softwood pulp with a lumen width (1) to fiber diameter (D) ratio (1/D) of 0.6 or lower is 40 percent by weight or higher, and the compounding quantity of a sizing agent is 0.05 percent by weight or higher but no higher than 0.12 percent by weight; and its density is 0.60 g/cm³ or lower.
2. The stool-receiving sheet for stool collection according to 1, characterized in that the compounding quantity of the softwood pulp is 50 percent by weight or higher.
3. The stool-receiving sheet for stool collection according to 1 or 2, characterized in that its density is 0.58 g/cm³ or lower.

### Effects of the Invention

The stool-receiving sheet for stool collection proposed by the present invention has excellent floatability on water so that, once it is floated on the water, it can remain floating when a sample is collected, without causing the stool to come in contact with the standing water. The stool-receiving sheet for stool collection proposed by the present invention has excellent dispersibility in water so that, once a sample has been collected, it can be flushed directly in water without causing the sewer pipe to clog.

### Brief Description of the Drawings

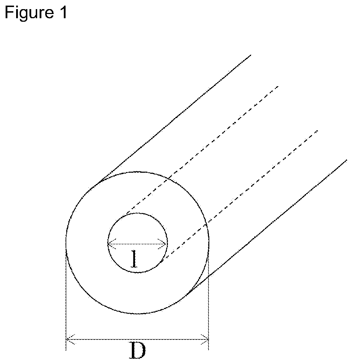

[FIG. 1] A schematic view of a cross-section of a pulp fiber.

### Mode for Carrying Out the Invention

### <Stool-Receiving Sheet for Stool Collection>

The stool-receiving sheet for stool collection proposed by the present invention (hereinafter also referred to as "stool-receiving sheet") is characterized in that: relative to the total quantity of papermaking fibers, the compounding quantity of a softwood pulp with a lumen width (1) to fiber diameter (D) ratio (1/D) of 0.6 or lower is 40 percent by weight or higher, and the compounding quantity of a sizing agent is 0.05 percent by weight or higher but no higher than 0.12 percent by weight; and its density is 0.60 g/cm³ or lower.

In this Specification, the lumen width (1) to fiber diameter (D) ratio (1/D) is hereinafter also referred to simply as "1/D."

### • 1/D

FIG. 1 shows a schematic view of a cross-section of a pulp fiber. What is generally referred to as a "pulp" is constituted primarily by tracheids in the case of a softwood pulp, or wood fibers in the case of a hardwood pulp, each having an inner cavity called "lumen" inside. The 1/D is a value indicating the percentage of size of the fiber occupied by the lumen, where the smaller this value, the smaller the lumen and the thicker the fiber wall.

Here, in this Specification, the 1/D of a pulp can be measured according to the procedure below:
1. Dilute a suspension of the pulp to a concentration of 0.05%.
2. Using a pipette, transfer a droplet of the diluted suspension onto a clean slide glass, and if necessary, distribute the fibers evenly using a dissecting needle.
3. Put a cover glass on top in such a way that air bubbles will not enter, and remove excess water using an absorbent paper, to produce a slide preparation.
4. Observe the slide preparation with an optical microscope and measure the fiber diameters (D) and lumen widths (1) by moving the slide preparation horizontally or vertically on the moving stage, to calculate the 1/Ds.

When preparing a paper material, the pulp to be compounded can be measured according to the above method to obtain the 1/Ds of at least 100 pulp fibers and use the average as the 1/D of the pulp.

When a paper has already been made, the paper can be defibrated in the same manner as that in preparing a slurry for measuring a defibration freeness of paper, after which a dye solution that manifests different colors according to the types of trees, etc., is used to discriminate the pulp type using at least 200 pulp fibers, while their 1/Ds are obtained according to the aforementioned method, thereby obtaining the compounding quantity of the softwood pulp and the 1/D of the pulp.

### • Papermaking Fibers

The stool-receiving sheet proposed by the present invention is characterized in that the compounding quantity of the softwood pulp with an 1/D of 0.6 or lower is 40 percent by weight or higher relative to the total quantity of papermaking fibers.

For the stool-receiving sheet proposed by the present invention, the softwood pulp with an 1/D of 0.6 or lower can be used in combination with other pulps, as papermaking fibers, so long as a paper of 0.60 g/cm³ or lower in density can be made. The compounding quantity of the softwood pulp with an 1/D of 0.6 or lower is preferably 50 percent by weight or higher, or more preferably 70 percent by weight or higher, relative to the total quantity of papermaking fibers.

### • Softwood Pulp with 1/D of 0.6 or Lower

The softwood pulp with an 1/D of 0.6 or lower floats easily on water because its fiber walls are thick and thus hardly collapse, and therefore it easily maintains a state in which air is trapped in the lumens and fiber voids. Also, the softwood pulp with an 1/D of 0.6 or lower disperses easily in water because its fiber walls are thick and thus hardly collapse, which allows a low-density sheet to be formed that has fewer fiber-to-fiber bonds. When the 1/D is lower, a sheet that is more easily floatable on water and more easily dispersible in water can be made, which means that, with regard to the softwood pulp with an 1/D of 0.6 or lower, its 1/D is preferably 0.55 or lower, or more preferably 0.5 or lower, or yet more preferably 0.45 or lower.

The softwood pulp with an 1/D of 0.6 or lower is not limited in any way, and any type of mechanical pulp (MP), thermo-mechanical pulp (TMP), sulfite pulp (SP), kraft pulp (KP), or bleached pulp (BP), or unbleached pulp (UP) based thereon, may be used alone or two or more types may be used in combination. Among these, a kraft pulp (KP) or sulfite pulp (SP) is preferred as they offer excellent strength.

### • Other Pulps

For the other pulps to be combined with the softwood pulp with an 1/D of 0.6 or lower, softwood pulps with an 1/D exceeding 0.6, hardwood pulps, and non-wood pulps such as recycled cellulose, flax, kenaf kozo, mitsumata, paper bush, and other bast fibers, bagasse, bamboo, esparto, and other hard fibers, cotton linter and other seed hair fibers, and manila hemp, sisal hemp, and other leaf vein fibers, may be used without any limitation, for example.

The 1/Ds of the other pulps are not limited in any way and may exceed 0.6. If the 1/Ds of the other pulps are high, however, the floatability and dispersibility on/in water tend to drop and therefore the compounding quantity of the softwood pulp with an 1/D of 0.6 or lower must be increased.

Preferably the papermaking fibers used in the stool-receiving sheet are beaten to a freeness (Canadian Standard Freeness: CSF) of 550 ml CSF or higher but no higher than 750 ml CSF. If their freeness drops below 550 ml CSF (due to further beating), the dispersibility in water will drop. If the freeness exceeds 750 ml CSF, the papermaking may become difficult. The freeness (Canadian Standard Freeness: CSF) is preferably 600 ml CSF or higher but no higher than 730 ml CSF, or more preferably 630 ml CSF or higher but no higher than 710 ml CSF.

The beating of pulp suspension is not limited to beating a suspension consisting only of the softwood pulp; instead, the softwood pulp and other pulps may be mixed and then beaten in a mixed state, or the softwood pulp and other pulps may be beaten separately and then mixed (separate beating). In the case of separate beating, the freeness of each pulp is not limited in any way; however, it should be adjusted so that the freeness of the pulp suspension obtained by mixing the respective pulps and using them for making the inner layer will fall in the aforementioned range of 550 ml CSF or higher but no higher than 750 ml CSF.

### • Sizing Agent

The stool-receiving sheet proposed by the present invention uses a sizing agent by a compounding quantity of 0.05 percent by weight or higher but no higher than 0.12 percent by weight in a bone-dry state. When the compounding quantity of the sizing agent is in the aforementioned range, the stool-receiving sheet proposed by the present invention can demonstrate floatability and dispersibility on/in water in a well-balanced manner. If the compounding quantity of the sizing agent is lower than 0.05 percent by weight, the floatability on water may drop. If the compounding quantity of the sizing agent exceeds 0.12 percent by weight, the dispersibility in water may drop. The compounding quantity of the sizing agent is more preferably 0.06 percent by weight or higher but no higher than 0.11 percent by weight, or yet more preferably 0.08 percent by weight or higher but no higher than 0.10 percent by weight. It should be noted that the compounding quantity of the sizing agent contained in the stool-receiving sheet can be measured according to the pyrolysis-gas chromatography-mass spectroscopy method.

For the sizing agent, a rosin, alkyl ketene dimer (AKD), alkenyl succinic anhydride (ASA), styrene-acrylic resin or other polymer-based sizing agent, higher fatty acid-based sizing agent, or any other known sizing agent may be added as an ingredient, or through external application, or both.

### • Additives

The stool-receiving sheet proposed by the present invention may, if necessary, have known additives selected and compounded in it as deemed appropriate to the extent that doing so does not hinder the floatability and dispersibility on/in water, such as polyacrylic amide-based polymer, polyvinyl alcohol-based polymer, cationized starch, any of various modified starches, urea-formalin resin, melamine-formalin resin, or other dry paper strengthening agent, polyamide polyamine epichlorohydrin resin, polyamine epichlorohydrin resin, polyamide epichlorohydrin resin, polyvinyl amine resin, polyethylene imine resin, or other wet paper strength enhancing agent, yield agent, drainage enhancing agent, coagulant, aluminum sulfate, bulking agent, dye, fluorescent whitening agent, pH adjusting agent, defoaming agent, UV protection agent, discoloration inhibitor, pitch controlling agent, slime controlling agent, and other additives.

### • Density

The stool-receiving sheet proposed by the present invention has a density of 0.60 g/cm³ or lower. When its density is 0.60 g/cm³ or lower, the sheet can remain floating on standing water in a toilet bowl, while bearing a stool on top, for sufficient time to complete the stool collection. The density is preferably 0.58 g/cm³ or lower, or more preferably 0.55 g/cm³ or lower, or yet more preferably 0.52 g/cm³ or lower.

### • Basis Weight

Preferably the basis weight of the stool-receiving sheet proposed by the present invention is 20 g/m² or higher but no higher than 60 g/m². If the basis weight is lower than 20 g/m², the floatability on water may become insufficient. If the basis weight is higher than 60 g/m², the dispersibility in water may drop. The basis weight is more preferably 30 g/m² or higher but no higher than 50 g/m², or yet more preferably 40 g/m² or higher but no higher than 45 g/m².

### • Floatability on Water

The floatability on water of the stool-receiving sheet proposed by the present invention can be evaluated based on the sinking time measured according to the method below.

### <Sinking Time>

Float the stool-receiving sheet (257 mm x 364 mm) on water, gently place at the center of the stool-receiving sheet an acrylic case (7.5 mm wide x 11.5 mm long x 44 mm high) containing a weight that brings the total weight of the case to 270 g, and measure the time it takes for one half the volume of the acrylic case to submerge in water, as the "sinking time."

With the stool-receiving sheet proposed by the present invention, this sinking time is preferably 180 seconds or longer, or more preferably 240 seconds or longer, or yet more preferably 300 seconds or longer. The longer the sinking time, the easier it is for the stool-receiving sheet to remain floating on water, thus demonstrating excellent floatability on water. For this reason, the time needed to complete the stool collection can be secured easily with the stool-receiving sheet having a long sinking time.

### • Dispersibility in Water

The dispersibility in water of the stool-receiving sheet proposed by the present invention can be evaluated based on the time of dispersion to a small floc state in water according to 4.5, "Ease of Disintegration" in JIS P4501. The time of dispersion to a small floc state in water indicates the time it takes for a 3-cm square test piece to tear into two or more pieces after the test piece is introduced into a 300-ml beaker containing 300 ml of deionized water and the water is stirred at 650 rpm with a stirrer.

The time of dispersion to a small floc state in water, of the stool-receiving sheet proposed by the present invention, is preferably within 120 seconds, or more preferably within 90 seconds, or yet more preferably within 60 seconds. Also, with the stool-receiving sheet proposed by the present invention, the ease of disintegration per 4.5 of JIS P4501 is preferably within 100 seconds, but not to exceed the upper limit of 200 seconds. If these times become longer, the dispersibility in water may drop and cause the plumbing, etc., to clog.

### <Manufacturing Method>

The stool-receiving sheet proposed by the present invention may be manufactured using any known art of papermaking. The paper machine may be of any type, such as cylinder paper machine, inclined short-Fourdrinier paper machine, Fourdrinier paper machine, or twin-wire paper machine, where any of these different machines may be used according to the required strength and dispersibility in water. For example, use of a cylinder paper machine associated with significant paper strength anisotropy, or specifically weaker strength in the horizontal direction than in the vertical direction, allows a paper to be manufactured that easily tears in the horizontal direction in water.

### Examples

The present invention is explained using examples below; it should be noted, however, that the present invention is not limited to the following examples.

### [Example 1]

To a softwood kraft pulp (1/D: 0.41) beaten to a Canadian Standard Freeness of 690 ml CSF, a sizing agent (manufactured by Arakawa Chemical Industries, Ltd., product name: Sizepine K-901) was added so that its compounding quantity would equal 0.08 percent by weight in a bone-dry state, and the mixture was put through a cylinder paper machine to manufacture a stool-receiving sheet with a basis weight of 43 g/m².

### [Example 2]

A stool-receiving sheet was manufactured in the same manner as in Example 1, except that the sizing agent was added so that its compounding quantity would equal 0.06 percent by weight.

### [Example 3]

A stool-receiving sheet was manufactured in the same manner as in Example 1, except that the sizing agent was added so that its compounding quantity would equal 0.10 percent by weight.

### [Example 4]

A stool-receiving sheet was manufactured in the same manner as in Example 1, except that 50 percent by weight of a softwood kraft pulp (1/D: 0.41) and 50 percent by weight of a hardwood kraft pulp, both beaten to a Canadian Standard Freeness of 620 ml CSF, were used.

### [Comparative Example 1]

A stool-receiving sheet was manufactured in the same manner as in Example 1, except that no sizing agent was added.

### [Comparative Example 2]

A stool-receiving sheet was manufactured in the same manner as in Example 1, except that a softwood kraft pulp (1/D: 0.70) beaten to a Canadian Standard Freeness of 690 ml CSF was used.

### [Comparative Example 3]

A stool-receiving sheet was manufactured in the same manner as in Example 1, except that the sizing agent was added so that its compounding quantity would equal 0.04 percent by weight.

### [Comparative Example 4]

A stool-receiving sheet was manufactured in the same manner as in Example 1, except that the sizing agent was added so that its compounding quantity would equal 0.20 percent by weight.

### [Comparative Example 5]

A stool-receiving sheet was manufactured in the same manner as in Example 1, except that 30 percent by weight of a softwood kraft pulp (1/D: 0.41) and 70 percent by weight of a hardwood kraft pulp, both beaten to a Canadian Standard Freeness of 600 ml CSF, were used.

The following evaluations were performed on the obtained stool-receiving sheets. The results are shown in Table 1.

### • Basis Weight, Thickness, Density

The basis weight was measured according to JIS P8124.

The thickness of one sheet was measured according to JIS P8118, based on a pressure of 100 kPa between the pressurized surfaces.

The density was calculated from the measured basis weight and thickness.

### • Sinking Time

The sinking time was measured according to the aforementioned method and evaluated according to the criteria below:
⊙ 300 seconds or longer
○ 180 seconds or longer but shorter than 300 seconds
× Shorter than 180 seconds

### • Ease of Disintegration

The ease of disintegration was measured according to 4.5, "Ease of Disintegration" in JIS P4501 and evaluated according to the criteria below:
○ 100 seconds or shorter
Δ Longer than 100 seconds but 200 seconds or shorter
× Longer than 200 seconds

### • Time of Dispersion to a Small Floc State in Water

The time of dispersion to a small floc state in water was measured according to the aforementioned method and evaluated according to the criteria below:
⊙ 60 seconds or shorter
○ Longer than 60 seconds but 120 seconds or shorter
× Longer than 120 seconds

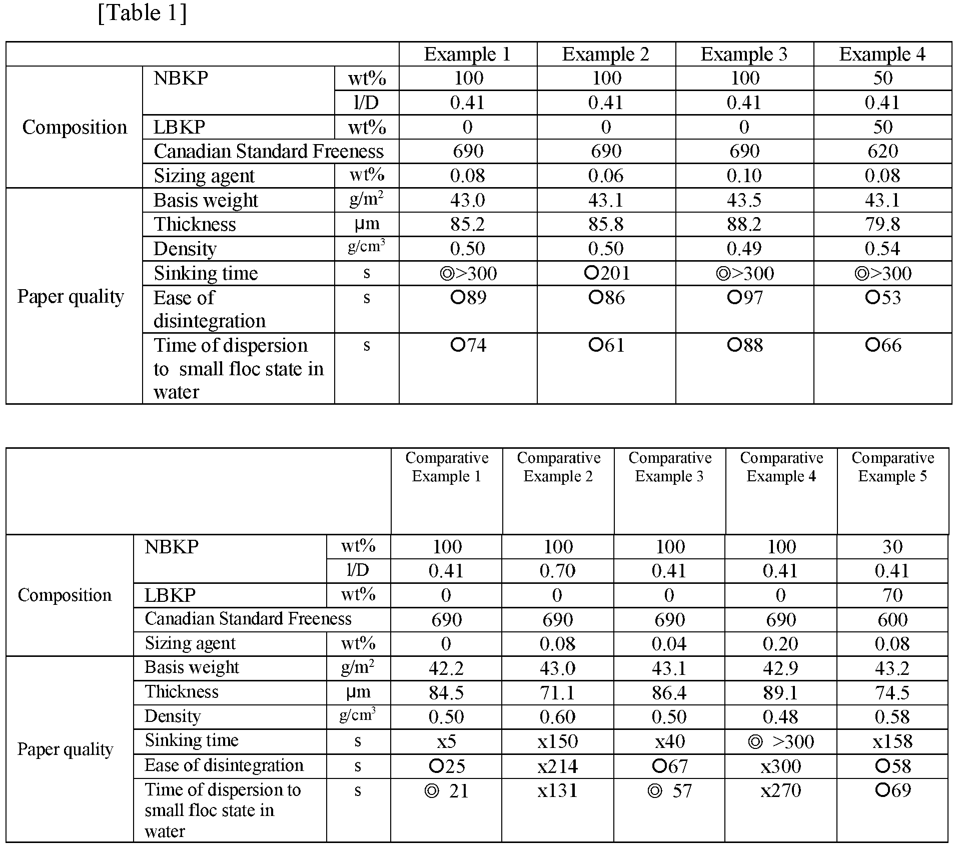

The stool-receiving sheets made in Examples 1 to 4, all of which conform to the present invention, demonstrated a long sinking time and excellent dispersibility in water and thus can be used favorably as stool-receiving sheets.

The stool-receiving sheets made in Comparative Examples 1 and 3 had excellent dispersibility in water, but their sizing property was poor and therefore when these sheets were floated on water, they sank quickly as the water permeated through to the top surface of the paper.

The stool-receiving sheet made in Comparative Example 2, wherein the 1/D of the softwood pulp used was higher than in Examples 1 to 4, did not float easily on water and showed poor dispersibility in water.

The stool-receiving sheet made in Comparative Example 4 had high sizing property and excellent floatability on water, but its dispersibility in water was low.

The stool-receiving sheet made in Comparative Example 5, which used a lesser quantity of softwood pulp with an 1/D of 0.6 or lower compared to the stool-receiving sheet made in Example 4, exhibited lower floatability on water.

## Claims

1. A stool-receiving sheet for stool collection, **characterized in that**:
relative to a total quantity of papermaking fibers, a compounding quantity of a softwood pulp with a lumen width (1) to fiber diameter (D) ratio (1/D) of 0.6 or lower is 40 percent by weight or higher, and a compounding quantity of a sizing agent is 0.05 percent by weight or higher but no higher than 0.12 percent by weight; and
a density is 0.60 g/cm³ or lower.

2. The stool-receiving sheet for stool collection according to claim 1, **characterized in that** the compounding quantity of the softwood pulp is 50 percent by weight or higher.

3. The stool-receiving sheet for stool collection according to claim 1 or 2, **characterized in that** its density is 0.58 g/cm³ or lower.
